# EUROPÄISCHE PATENTSCHRIFT

(11) **EP 1 480 942 B1**
(45) Veröffentlichungstag und Bekanntmachung des Hinweises auf die Patenterteilung: **29.04.2009**
(21) Anmeldenummer: 03704600.0
(22) Anmeldetag: 13.02.2003
(51) Int. Cl.: C07C 209/48, B01J 25/02, B01J 37/02

(54) **VERFAHREN ZUR HERSTELLUNG VON PRIMÄREN AMINEN DURCH HYDRIERUNG VON NITRILEN**
METHOD FOR THE PRODUCTION OF PRIMARY AMINES BY HYDROGENATING NITRILES
PROCEDE POUR PRODUIRE DES AMINES PRIMAIRES PAR HYDROGENATION DE NITRILES

(30) Priorität: 23.02.2002 DE 10207926
(43) Veröffentlichungstag der Anmeldung: 01.12.2004
(73) Patentinhaber: Clariant Produkte (Deutschland) GmbH, 65929 Frankfurt am Main (DE)
(72) Erfinder: GÖBÖLÖs, Sandor, H-1022 Budapest (HU); FASI, Andras, H-2400 Dunaujvaros (HU); MARGITFALVI, Jozsef, H-1221 Budapest (HU); MILLIAN, Laszlone, H-1204 Budapest (HU)
(86) Internationale Anmeldenummer: PCT/EP2003/001407
(87) Internationale Veröffentlichungsnummer: WO 2003/070688

(56) Entgegenhaltungen:
- US-A- 3 544 485
- US-A- 3 980 720
- US-A- 4 876 362
- DATABASE WPI Week 200028, 26. Januar 2000 (2000-01-26) Derwent Publications Ltd., London, GB; AN 2000-318608 XP002240818 & CN 1 242 260 A (HUBEI PROV CHEMICAL RESEARCH I (CN) )

## Beschreibung

Die vorliegende Erfindung betrifft die Herstellung von primären Aminen durch katalytische Hydrierung von Nitrilen.

Der Hydrierung von Nitrilen zu Aminen kommt aufgrund der weitreichenden Anwendungen von Aminen, wie z.B. als organische Lösungsmittel, Agrochemikalien, Pharmazeutika, Tenside und insbesondere Zwischenprodukt von Polyamid-6,6 große technische Bedeutung zu. Die Hydrierung erfolgt in der Regel an Raney-Nickel-Katalysatoren in flüssiger Phase bei erhöhten Temperaturen und Wasserstoffdrücken, wobei zur Erhöhung der Ausbeute des primären Amins durch Inhibierung der Bildung der sekundären und tertiären Amine Ammoniak zugegen ist.

Die Hydrierung von Nitrilen zu primären Aminen wird beispielsweise in den folgenden Patentschriften und Aufsätzen erläutert.

E.J. Schwoegler und H. Adkins, J. Am. Chem. Soc. 61, 3499 (1939) sagten voraus und konnten dann auch zeigen, dass durch Zusatz von genügend Ammoniak bei der Hydrierung eines Nitrils die Bildung von sekundärem Amin stark inhibiert und somit die Selektivität zu primärem Amin stark verbessert wird.

In US-2 165 515 wird ein Verfahren zur Herstellung von primären Aminen durch katalytische Hydrierung von Nitrilen unter Verwendung von Cobalt und mit Barium oder Mangan promotiertem Cobalt beschrieben.

S. Sakakibara et al., J. Chem. Soc. Japan, Ind. Chem. Sect., 56, 497 (1953), beschreiben ein Verfahren zur Herstellung von primären Aminen wie Dodecylamin durch Hydrierung von Nitrilen wie Lauronitril mit Ammoniak unter Verwendung von Raney-Nickel-Katalysatoren.

In US-3 574 754 wird ein Verfahren zur Herstellung von primären Aminen durch Hydrierung von Nitrilen mit Ammoniak unter Verwendung von Raney-Nickel-Katalysatoren beschrieben.

In US-4 739 120 wird ein Verfahren zur Hydrierung von Nitrilen zu primärem Amin unter Verwendung eines Rhodium-Katalysators beschrieben. Die Umsetzung wird in Gegenwart eines zweiphasigen Lösungsmittelsystems mit einer wässrigen Phase und einer nicht wassermischbaren organischen Phase durchgeführt.

M. Besson et al., Stud. Surf. Sci. Catal., Band 59, "Heterogeneous Catalysis and Fine Chemicals II", Hrsg. M. Guisnet et al., 1991, S. 113-120, offenbaren ein Verfahren zur Herstellung von primären Aminen aus Nitrilen in Gegenwart von Cyclohexan unter Verwendung eines mit Molybdän oder Chrom dotierten Raney-Nickel-Katalysators.

In EP-A-0 547 505 wird ein Verfahren zur Herstellung von Dodecylamin durch katalytische Hydrierung von Lauronitril in Gegenwart von Ammoniak unter Verwendung eines auf einem Träger copräzipitierten Magnesium-Nickel-Katalysators beschrieben.

In PL-51 350 wurde zur Hydrierung von aliphatischen Nitrilen und insbesondere Lauronitril ein Nickel-Katalysator eingesetzt. Zur Unterdrückung der Produktion von sekundärem Amin war Ammoniak zugegen.

In JP-A-7-941 804 wird ein Verfahren zur Herstellung von primären Aminen durch Hydrierung des Nitrils in Mischungen aus niederem Alkohol und cyclischem Kohlenwasserstoff in Gegenwart von mit Alkali- oder Erdalkalimetallhydroxiden und Chrom modifiziertem Raney-Nickel-Katalysator beschrieben.

In JP-A-6-032 767 wird ein Verfahren zur Hydrierung von Nitrilen zu primärem Amin unter Verwendung eines Raney-Nickel-Katalysators in Gegenwart von Ammoniak beschrieben.

D. Djaouadi et al., Catal. Org. Reactions, 423 (1995), stellten durch alkalisches Auslaugen von intermetallischen Legierungen ausgehend von Ni₄₀Al₆₀ oder Ni₂₅Al₇₅ einen mit Chrom und Eisen dotierten Raney-Nickel-Katalysator her. Die selektive Hydrierung von Valeronitril zum entsprechenden primären Amin wurde in Cyclohexan in einem Rührautoklaven durchgeführt. Die mit der Dotierung erzielte Verbesserung der Selektivität zum primären Amin wird hauptsächlich durch eine Abnahme der Hydrierungsrate des als Zwischenprodukt entstehenden sekundären Imins, durch die Rückreaktionen begünstigt werden, erklärt.

S. Xie et al., Appl. Catal. A: General, 189, 45 (1999) berichteten, dass durch das Ammoniakadditiv die Selektivität zum primären Amin bei der selektiven Hydrierung von Stearonitril an amorphen Ni-B/SiO₂-Katalysatoren stark verbessert wurde, wenngleich eine geringfügige Abnahme der Aktivität beobachtet wurde. Die amorphe Struktur des Katalysators und des zulegierten Bors spielten Schlüsselrollen bei der Förderung der Hydrieraktivität und der Selektivität zu primären Aminen. Bei mit KNO₃ dotiertem SiO₂ wurde jedoch kein wesentlicher Promotionseffekt beobachtet.

Zur Verbesserung der Selektivität bei der Hydrierung von organischen Nitrilen zu Aminen wurden auch schon Alkalimetallhydroxid-Additive eingesetzt. Laut T.A. Johnson et al., Proceedings of the "18th Conference on Catalysis of Organic Reactions", Hrsg. M. E. Ford, 2000, Aufsatz 13, konnte die selektive Nitrilhydrierung unter Verwendung von mit Lithiumhydroxid modifizierten Nickel- und Cobaltschwammkatalysatoren durchgeführt werden.

In US-5 874 625 wird ein Verfahren zur katalytischen Hydrierung von organischen Nitrilen zu primären Aminen unter Verwendung einer Aufschlämmung von Raney-Nickel-Katalysator und wässrigem Alkalimetallhydroxid beschrieben, wobei der Raney-Nickel-Katalysator und das wässrige Alkalimetallhydroxid etwa 0,1 bis etwa 0,3 % Wasser beitragen. Die Autoklavencharge wird mit Wasserstoff beaufschlagt und dann auf eine. Endtemperatur von etwa 110°C erhitzt.

In US-5 777 166 wird ein Verfahren zur Hydrierung von Nitrilen zu Aminen beschrieben, bei dem man: a) einen Katalysator vom Raney-Nickel-Typ mit mindestens einem zusätzlichen Metallelement aus der Gruppe IVb des Periodensystems der Elemente dotiert, wobei man von einer metallurgischen Ni/Al/Dotierelement-Vorläuferlegierung ausgeht und das Dotierelement/Ni-Gewichtsverhältnis zwischen 0,05 und 10% liegt; und b) auf den Katalysator ein Nitril in einem flüssigen Reaktionsmedium, in dem das Nitril löslich ist, zusammen mit mindestens einer anorganischen Base aus der Gruppe bestehend aus LiOH, NaOH, KOH, RbOH und CsOH einwirken lässt und dadurch das Nitril hydriert.

In US-5 869 653 wird ein Verfahren zur katalytischen Hydrierung von Nitrilen beschrieben, bei dem man das Nitril in Gegenwart eines Cobaltschwammkatalysators unter ammoniakfreien Bedingungen mit Wasserstoff in Berührung bringt, um die Nitrilgruppe in das primäre Amin umzuwandeln, wobei die Verbesserung des Hydrierverfahrens darin besteht, dass man die Hydrierung in Gegenwart eines mit einer katalytisch wirksamen Menge Lithiumhydroxid behandelten Cobaltschwammkatalysators durchführt und die Umsetzung in Gegenwart von Wasser durchführt.

WO-01/66511 offenbart ein Verfahren zur Hydrierung von Nitrilen zu Aminen, wobei allerdings keine Modifizierung des Hydrierkatalysators vor der Hydrierung stattfindet.

Zur Förderung der Selektivität von Raney-Nickel-Katalysatoren für die Herstellung von Aminen bei der Alkylierung von Ammoniak oder Alkylaminen mit Alkoholen wurden auch schon Alkalimetallcarbonate eingesetzt. In FR-A-2 351 088, DE-A-26 21 449, DE-A-26 25 196 und DE-A-26 39 648 wird die Herstellung von tertiären Aminen durch Alkylierung von sekundären Aminen mit Alkoholen unter Wasserabspaltung in der flüssigen Phase in Gegenwart eines Hydrierungs-Dehydrierungskatalysators wie Raney-Nickel oder Raney-Cobalt und einer oder mehreren basischen Alkalimetall- oder Erdalkalimetallverbindungen beschrieben. So wurde eine Mischung aus 774 kg n-Dodecanol, 500 kg Ethylbenzol, 10 kg

Na₂CO₃ und 300 kg Raney-Nickel auf 130-5°C erhitzt und unter Auskreisen von Wasser mit Wasserstoff und Dimethylamin beschickt, bis die theoretische H₂O-Menge abgeschieden worden war, wonach der Katalysator abfiltriert und das Filtrat destilliert wurde, was 80 % RNMe₂, 15 % R₂NMe und 5 % R₃N (R = n-Dodecyl) ergab.

In DE-A-26 45 712 wird ein Verfahren zur Herstellung von sekundären Aminen durch Alkylierung von Ammoniak mit Alkoholen in Gegenwart eines Hydrierungs-Dehydrierungskatalysators und einer basischen Alkali- oder Erdalkalimetallverbindung beschrieben. So wurde ein Dephlegmator-Reaktor mit Stearylalkohol, Raney-Nickel und Na₂CO₃ bei 90-140°C unter kontinuierlicher Entfernung von H₂O bei atmosphärischem Druck mit Ammoniak beschickt, was > 95 % Distearylamin ergab.

M. Kalina und Yu. Pashek (Kinetika i Kataliz, 10, 574, 1969) berichteten über die Verwendung von mit Na₂CO₃ modifizierten Cobalt- und Nickelkatalysatoren für die Flüssigphasenhydrierung von Palmitinsäurenitril. Die Zugabe von Natriumcarbonat zum Reaktionsgemisch in einem Na₂CO₃/Katalysator/Nitril-Gewichtsverhältnis von 5/5/100 bei 150°C und 50 bar Wasserstoffdruck ergab eine Abnahme der Selektivität zum sekundären Amin. Die bei 50 % Umsatz bestimmte Selektivität zum sekundären Amin betrug an Co-, Ni-, Co+Na₂CO₃-, Ni+Na₂CO₃-Katalysatoren 17,4, 20,1, 11,0 bzw. 12,2 %.

Zur Herstellung von unsymmetrischen aliphatischen sekundären Alkylaminen wurde ein geträgertes Nickel und Alkalicarbonat enthaltendes Katalysatorsystem verwendet. In US-5 254 736 und EP-A-0 526 318 wird ein Verfahren zur Herstellung von sekundären Methylalkylaminen der allgemeinen Formel R-NH-CH₃, worin R für eine aliphatische C₁₀-C₂₂-Kette steht, durch Aminierungsreaktion zwischen einem Alkohol und einem Monoalkylamin beschrieben. Die Aminierungsreaktion wurde mit einem Nickel-Trägerkatalysator in Gegenwart eines Alkalicarbonats, am besten Kaliumcarbonat, bei einem Kaliumcarbonat/Nickelkatalysator-Gewichtsverhältnis zwischen 1:4 und 1:1 unter einem Wasserstoffdruck zwischen 10 und 50 bar durchgeführt. In einem typischen Beispiel wurden 422 g Dodecylamin (2,3 mol), 1460 g Methanol (45 mol), 38,6 g Nickel-Trägerkatalysator (Harshaw Ni 1404T) und 57,1 g K₂CO₃ bei 180°C, 40 bar Wasserstoffdruck und 1800 UpM 6 Stunden autoklaviert. Das Reaktionsprodukt setzte sich aus 0 % RNH₂, 85,9 % RNHMe, 1,3 % RNMe₂, 6,0 % R₂NH und 6,8 % ROH zusammen, wohingegen sich die Konzentrationen am unmodifizierten Katalysator auf 15,0 %, 17,2 %, 44,9 %, 13,8 % bzw. 9,1 % beliefen (R = n-Dodecyl, Me = Methyl).

Die im Stand der Technik vorbeschriebenen Verfahren zur Herstellung von primären Aminen durch Hydrierung von Nitrilen sind mit den folgenden Nachteilen behaftet:
(i) Zur Verringerung der Selektivität zu sekundären und tertiären Aminen muss Ammoniak verwendet werden.
(ii) Trotz der durch die Zugabe eines Alkalimetallhydroxids oder -carbonats erreichten verbesserten Selektivität zu primärem Amin kann die kontrollierte und reproduzierbare Wechselwirkung zwischen dem Additiv und dem Katalysator nicht garantiert werden.
(iii) Sofern die als Additiv verwendete Alkalimetallverbindung in dem Katalysator vergleichbarer Menge eingesetzt wird, ist die Gegenwart einer bestimmten Menge Wasser im Reaktionsgemisch erforderlich. Dadurch werden die Wiederverwendung des Katalysators und die Trennung von Reaktionsprodukten erschwert.

Die Nachteile (ii) und (iii) betreffen auch das Verfahren zur Herstellung von sekundärem Amin aus einem Alkohol und einem primären Amin an einem Nickel-Trägerkatalysator und in Gegenwart einer großen Menge an dem Reaktionsgemisch zugesetztem Alkalimetallcarbonat (US-5 254 736 und EP-0 526 318).

Dieser Erfindung lag die Aufgabe zugrunde, die mit den vorbeschriebenen Verfahren zur Herstellung von primären Aminen durch katalytische Hydrierung von Nitrilen verbundenen Nachteile zu überwinden. Die Aufgabe besteht somit in der Unterdrückung der Bildung von sekundärem und tertiärem Amin bei der Hydrierung von Nitrilen zu primären Aminen. Die Erfindung zielt ferner darauf ab, auf Ammoniak zu verzichten oder dessen Partialdruck bei der Nitrilhydrierung möglichst gering zu halten.

Überraschenderweise wurde gefunden, dass bei der Hydrierung von Nitrilen, z.B. Fettsäurenitrilen wie Lauronitril, in Abwesenheit von Ammoniak Alkalimetallcarbonat oder -hydrogencarbonat, insbesondere K₂CO₃, der beste Modifikator für den Hydrierkatalysator zur Unterdrückung der Selektivität zum sekundären Amin auf höchstens 2 % ist. Die geringe Selektivität zum sekundären Amin wird mit Alkalimetallcarbonat oder -hydrogencarbonat bis zu einem Nitril-Umsatz von 99 % aufrechterhalten. Die Modifizierung des Hydrierkatalysators mit dem Alkalimetallcarbonat oder -hydrogencarbonat, wie K₂CO₃ oder KHCO₃ kann in einer Aufschlämmung unter Verwendung von destilliertem Wasser als Lösungsmittel durchgeführt werden.

Gegenstand der vorliegenden Erfindung ist somit ein Verfahren zur Herstellung eines primären Amins durch Hydrierung von Nitrilen, bei dem man ein Reaktionsgemisch, das
(a) mindestens ein Nitril,
(b) Wasserstoff,
(c) gegebenenfalls Ammoniak und
(d) mindestens einen, durch Adsorption eines Alkalimetallcarbonats oder Alkalimetallhydrogencarbonats ex situ modifizierten, Cobalt- oder Nickelkatalysator enthält, welcher Alkalimetallcarbonat oder - hydrogencarbonat in einer Menge von 2 bis 12 Gew.-% enthält,
umsetzt.

Ein weiterer Gegenstand der Erfindung ist ein modifizierter Cobalt- oder Nickelkatalysator, erhältlich durch Adsorption eines Alkalimetallcarbonats oder Alkalimetallhydrogencarbonats auf einen üblichen Cobalt- oder Nickelkatalysator. Besonders bevorzugt sind Raney-Nickel-Katalysatoren.

Die Modifizierung des Katalysators erfolgt mit Alkalimetallcarbonat oder -hydrogencarbonat. Als Alkalimetalle kommen Na, K, Rb, Cs in Frage. Bevorzugt sind die Alkalicarbonate, sowie insbesondere K₂CO₃ oder KHCO₃. Die Adsorption erfolgt aus einer Lösung der Alkalimetallcarbonate oder -hydrogencarbonate mit Konzentrationen von 10 g/l bis 400 g/l. Bevorzugt ist die Adsorption aus wässriger Lösung, insbesondere wässriger Lösung mit Konzentrationen von 50 bis 200 g/l.

Unter ex situ ist hier zu verstehen, dass der Katalysator außerhalb, insbesondere zeitlich vor, der Hydrierungsreaktion vom Nitril zum Amin, die durch ihn katalysiert wird, modifiziert wurde.

Das Gewichtsverhältnis von trockenem Katalysator zur Lösung von Alkalimetallcarbonat oder -hydrogencarbonat beträgt vorzugsweise 50 bis 350 g/l.

Bei dem unbehandelten Katalysator handelt es sich vorzugsweise um Cobalt, Nickel, Raney-Cobalt oder Raney-Nickel. Die Katalysatoren können ohne oder mit Promotoren verwendet werden. Promotoren sind beispielsweise Fe, Mo, Cr, Ti, Zr. Die Katalysatoren können auf Trägermaterialien aufgebracht sein. Solche Trägermaterialien sind beispielsweise SiO₂, Al₂O₃, ZrO₂, MgO, MnO, ZnO, Cr₂O₃.

Besonders bevorzugte Ausführungsformen sind
- Raney-Nickel ohne Promotoren, oder mit den Promotoren Fe, Mo, Cr, Ti, Zr
- Nickel auf den Trägermaterialien SiO₂, Al₂O₃, ZrO₂, MgO, MnO, ZnO, Cr₂O₃,
- Raney-Cobalt ohne Promotoren, oder mit den Promotoren Ni, Cr
- Cobalt auf den Trägermaterialien SiO₂, Al₂O₃, MgO, MnO.

Der unbehandelte Katalysator wird vorzugsweise in der Lösung von Alkalimetallcarbonat oder -hydrogencarbonat aufgeschlämmt und unter Wasserstoff oder einem Inertgas wie z.B. Stickstoff etwa 1 bis 16 Stunden gerührt.

Der Überschuss der Lösung wird nach der Adsorption durch Filtrieren unter Inertgas, vorzugsweise Stickstoffatmosphäre entfernt. Es ist vorteilhaft, den erhaltenen Katalysator nach der Adsorption mit Alkohol und danach mit einem Kohlenwasserstoff zu waschen.

Der modifizierte Katalysator wird in einer bevorzugten Ausführungsform durch Dekantieren und anschließendes dreimaliges Waschen des Katalysators mit Ethanol und zweimaliges Waschen mit Cyclohexan aus der Aufschlämmung gewonnen.

Das Alkalimetallcarbonat oder -hydrogencarbonat liegt in dem modifizierten Katalysator in einer Menge von 2 bis 12 Gew.-% vor. In einer besonders bevorzugten Ausführungsform enthält der modifizierte Katalysator K₂CO₃ oder KHCO₃ in einer Menge von 6 bis 7 Gew.-%.

Das erfindungsgemäße Verfahren eignet sich zur Hydrierung beliebiger Nitrile. Vorzugsweise handelt es sich um Nitrile der Formel R-CN, wobei R für eine gesättigte oder ungesättigte Kohlenwasserstoffgruppe von 1 bis 32, vorzugsweise 4 bis 24, insbesondere 8 bis 22 Kohlenstoffatomen steht. R steht weiterhin vorzugsweise für eine Alkylgruppe, insbesondere lineare Alkylgruppe.
Der modifizierte Katalysator liegt im Reaktionsgemisch in der Regel in der Menge von etwa 1 bis 10 Gew.-%, bezogen auf das Nitril, vor.

Das Reaktionsgemisch kann auch ein Lösungsmittel enthalten. Hierfür eignen sich vorzugsweise kurzkettige Alkohole, insbesondere Methanol, Ethanol und Propanol, sowie Kohlenwasserstoffe, wie Hexan, Cyclohexan und Toluol. Das Lösungsmittel kann in dem Gemisch in Mengen von etwa 0 bis 90 Gew.-%, bezogen auf das Reaktionsgemisch, vorliegen.

Wird dem Reaktionsgemisch Ammoniak zugesetzt, so sollte die Ammoniakmenge in dem Reaktionsgemisch 1 bis 10 Gew.-%, bezogen auf das Nitril, betragen.

Die Nitrilhydrierung an dem modifizierten Katalysator wird vorzugsweise unter einem Wasserstoffdruck von 1 bis 200, insbesondere 2 bis 30 bar durchgeführt.

Die Nitrilhydrierung an dem modifizierten Katalysator wird vorzugsweise im Temperaturbereich von 60 bis 250°C, insbesondere 100 bis 150°C durchgeführt.

### Beispiele

### Katalysatormodifizierung

Bei den Katalysatoren der Beispiele 1 bis 14 handelt es sich um Raney-Nickel-Katalysatoren.

### Beispiel 1

2 g Kaliumcarbonat wurden in 20 ml destilliertem Wasser (Konzentration c = 100 g/l) gelöst, und in der Lösung wurden 1,4 g wasserfeuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Kaliumgehalt des Katalysators betrug 2,4 Gew.-%.

### Beispiel 2

3 g Kaliumcarbonat wurden in 20 ml destilliertem Wasser (c = 150 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Kaliumgehalt des Katalysators betrug 3,2 Gew.-%.

### Beispiel 3

4 g Kaliumcarbonat wurden in 20 ml destilliertem Wasser (c = 200 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Kaliumgehalt des Katalysators betrug 4,1 Gew.-%.

### Beispiel 4

8 g Kaliumcarbonat wurden in 20 ml destilliertem Wasser (c = 400 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Kaliumgehalt des Katalysators betrug 4,6 Gew.-%.

### Beispiel 5

3 g Kaliumcarbonat wurden in 20 ml destilliertem Wasser (c = 150 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert. Der Kaliumgehalt des Katalysators betrug 7,1 Gew.-%.

### Beispiel 6

3 g Kaliumcarbonat wurden in 20 ml destilliertem Wasser (c = 150 g/l) gelöst, und in der Lösung wurden 1,4 g feuchter (1 g trockener) Katalysator suspendiert und 1 Stunde bei Raumtemperatur gerührt. Nach der Modifizierung wurde die Suspension unter Stickstoffatmosphäre filtriert. Der Kaliumgehalt des Katalysators betrug 3,2 Gew.-%.

### Beispiel 7

3 g Kaliumcarbonat wurden in 20 ml destilliertem Wasser (c = 150 g/l) gelöst, und in der Lösung wurden 1,4 g feuchter (1 g trockener) Katalysator suspendiert und unter Stickstoffatmosphäre 16 Stunden bei Raumtemperatur gerührt. Nach der Modifizierung wurde die Suspension dekantiert und der Katalysator dreimal mit 20 ml Ethanol und zweimal mit 20 ml Cyclohexan gewaschen. Der Kaliumgehalt des Katalysators betrug 3,3 Gew.-%.

### Beispiel 8

1,3 g Kaliumcarbonat wurden in 5 ml destilliertem Wasser (c = 150 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Kaliumgehalt des Katalysators betrug 3,1 Gew.-%.

### Beispiel 9

4 kg Kaliumcarbonat wurden in 28 I destilliertem Wasser (c = 143 g/l) gelöst, und in der Lösung wurden 2 kg feuchter (1,4 kg trockener) Katalysator suspendiert und unter Stickstoffatmosphäre 2 Stunden bei Raumtemperatur gerührt. Nach 20 Stunden setzte sich der Katalysator ab, und der Überschuss an Kaliumcarbonatlösung wurde im Vakuum abgezogen. Der Katalysator wurde unter einer dünnen Schicht Kaliumcarbonatlösung gelagert. Vor der Verwendung wurde die Suspension dekantiert und der Katalysator dreimal mit 20 ml Ethanol und zweimal mit 20 ml Cyclohexan gewaschen. Der Kaliumgehalt des Katalysators betrug 3,8 Gew.-%.

### Beispiel 10

140 g Kaliumcarbonat wurden in 610 ml destilliertem Wasser (c = 229 g/l Alkalimetallcarbonatlösung) gelöst, und in der Lösung wurden 300 g feuchter (210 g trockener) Katalysator suspendiert und unter Stickstoffatmosphäre 2 Stunden bei Raumtemperatur gerührt. Nach der Modifizierung setzte sich der Katalysator über Nacht ab, und der Überschuss an Lösung wurde im Vakuum abgezogen. Der modifizierte Raney-Nickel-Katalysator wurde unter Stickstoff abfiltriert. Der Kaliumgehalt des Katalysators betrug 3,9 Gew.-%.

### Beispiel 11

3 g Kaliumhydrogencarbonat wurden in 20 ml destilliertem Wasser (c = 150 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Kaliumgehalt des Katalysators betrug 2,3 Gew.-%.

### Beispiel 12

3 g Natriumcarbonat wurden in 20 ml destilliertem Wasser (c = 150 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Natriumgehalt des Katalysators betrug 2,4 Gew.-%.

### Beispiel 13

3 g Rubidiumcarbonat wurden in 20 ml destilliertem Wasser (c = 150 g/l) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Rubidiumgehalt des Katalysators betrug 4,8 Gew.-%.

### Beispiel 14

3 g Cäsiumcarbonat wurden in 20 ml destilliertem Wasser (c = 150 g/l Alkalimetallcarbonatlösung) gelöst, und in der Lösung wurden 1,4 g feuchter (1 g trockener) Katalysator suspendiert und 1 Stunde bei Raumtemperatur gerührt. Nach der Modifizierung wurde die Suspension dekantiert und der Katalysator dreimal mit 20 ml Ethanol und zweimal mit 20 ml Cyclohexan gewaschen. Der Cäsiumgehalt des Katalysators betrug 5,9 Gew.-%.

### Beispiel 15

30 g Kaliumcarbonat wurden in 200 ml destilliertem Wasser (c = 150 g/l) gelöst. In der Lösung wurden 10 g trockener Nickelkatalysator auf SiO₂ (Kieselgur)/MgO (pulverförmiger Ni 55/5 TS von Celanese) suspendiert und 1 Stunde bei Raumtemperatur gerührt. Nach der Modifizierung wurde die Suspension dekantiert, der Katalysator fünfmal mit 20 ml Ethanol gewaschen und im Vakuum getrocknet. Der Kaliumgehalt des Katalysators betrug 3 Gew.-%.

### Beispiel 16

30 g Kaliumcarbonat wurden in 200 ml destilliertem Wasser (c = 150 g/l) gelöst. In der Lösung wurden 10 g trockener Nickelkatalysator auf Kieselgur/Al₂O₃ (pulverförmiger Ni 62/15 TS von Celanese) suspendiert und 1 Stunde bei Raumtemperatur gerührt. Nach der Modifizierung wurde die Suspension dekantiert, der Katalysator fünfmal mit 20 ml Ethanol gewaschen und im Vakuum getrocknet. Der Kaliumgehalt des Katalysators betrug 3,5 Gew.-%.

### Beispiel 17

30 g Kaliumcarbonat wurden in 200 ml destilliertem Wasser (c = 150 g/l) gelöst. In der Lösung wurden 10 g trockener Cobaltkatalysator auf Kieselgur (pulverförmiger Co 45/20 TS von Celanese) suspendiert und 1 Stunde bei Raumtemperatur gerührt. Nach der Modifizierung wurde die Suspension dekantiert, der Katalysator fünfmal mit 20 ml Ethanol gewaschen und im Vakuum getrocknet. Der Kaliumgehalt des Katalysators betrug 3,3 Gew.-%.

### Beispiel 18

30 g Kaliumcarbonat wurden in 200 ml destilliertem Wasser (c = 150 g/l) gelöst. In der Lösung wurden 14 g wasserfeuchter Raney-Cobaltkatalysator B 2112 Z suspendiert und 1 Stunde bei Raumtemperatur gerührt. Nach der Modifizierung wurde die Suspension dekantiert und der Katalysator unter Stickstoff druckfiltriert. Der Kaliumgehalt des Katalysators betrug 3 Gew.-%.

**Tabelle 1: modifizierte Katalysatoren**

| Beispiel | Modifizierungsmittel | Katalysator | Alkalimetallgehalt des Katalysators, Gew.-% |
|---|---|---|---|
| 1 | Kaliumcarbonat | Raney-Nickel | 2,4 |
| 2 | Kaliumcarbonat | Raney-Nickel | 3,2 |
| 3 | Kaliumcarbonat | Raney-Nickel | 4,1 |
| 4 | Kaliumcarbonat | Raney-Nickel | 4,6 |
| 5 | Kaliumcarbonat | Raney-Nickel | 7,1 |
| 6 | Kaliumcarbonat | Raney-Nickel | 3,2 |
| 7 | Kaliumcarbonat | Raney-Nickel | 3,3 |
| 8 | Kaliumcarbonat | Raney-Nickel | 3,1 |
| 9 | Kaliumcarbonat | Raney-Nickel | 3,8 |
| 10 | Kaliumcarbonat | Raney-Nickel | 3,9 |
| 11 | Kaliumhydrogencarbonat | Raney-Nickel | 2,3 |
| 12 | Natriumcarbonat | Raney-Nickel | 2,4 |
| 13 | Rubidiumcarbonat | Raney-Nickel | 4,8 |
| 14 | Cäsiumcarbonat | Raney-Nickel | 5,9 |
| 15 | Kaliumcarbonat | Nickel | 3 |
| 16 | Kaliumcarbonat | Nickel | 3,5 |
| 17 | Kaliumcarbonat | Cobalt | 3,3 |
| 18 | Kaliumcarbonat | Raney-Cobalt | 3 |

### Nitrilhydrierung

### Vergleichsbeispiel 19 (nicht erfindungsgemäß)

In einem 300-ml-Reaktor aus rostfreiem Stahl wurden 100 ml (0,447 mol) Lauronitril und 1,4 g feuchter unmodifizierter Raney-Nickel-Katalysator vorgelegt. Es wurde unter 10 bar Wasserstoffdruck bei 125°C 2 Stunden umgesetzt, wobei das Reaktionsgemisch mit 1500 UpM gerührt wurde. Die Dodecylamin-Ausbeute betrug 83,2 % bei einem Umsatz von 99,7 %.

### Vergleichsbeispiel 20 (nicht erfindungsgemäß)

In dem Reaktor wurden 100 ml (0,447 mol) Lauronitril, 1,4 g feuchter unmodifizierter Raney-Nickel-Katalysator und 2,28 g (0,134 mol) Ammoniak vorgelegt. Es wurde unter 10 bar Wasserstoffdruck bei 125°C 4 Stunden umgesetzt, wobei das Reaktionsgemisch mit 1500 UpM gerührt wurde. Die Dodecylamin-Ausbeute betrug 93,6 % bei einem Umsatz von 99,8 %.

### Beispiel 21

In einem Reaktor wurden 100 ml Lauronitril und 1,4 g feuchter, mit 150 g/l K₂CO₃-Lösung gemäß Beispiel 2 modifizierter Raney-Nickel-Katalysator vorgelegt. Es wurde unter 10 bar Wasserstoffdruck bei 125°C 2 Stunden umgesetzt, wobei das Reaktionsgemisch mit 1500 UpM gerührt wurde. Die Dodecylamin-Ausbeute betrug 97,2 % bei einem Umsatz von 99,8 %.

### Beispiel 22

In dem Reaktor wurden 100 ml Lauronitril, 1,4 g mit 150 g/l K₂CO₃-Lösung modifizierter Raney-Nickel-Katalysator (Beispiel 2) und 2,28 g (0,134 mol) Ammoniak vorgelegt. Es wurde unter 10 bar Wasserstoffdruck bei 125°C 2 Stunden umgesetzt, wobei das Reaktionsgemisch mit 1500 UpM gerührt wurde. Die Dodecylamin-Ausbeute betrug 99,4 % bei einem Umsatz von 99,6 %.

### Beispiel 23

Es wurde analog Beispiel 21 verfahren, jedoch mit der Abwandlung, dass der Raney-Nickel-Katalysator mit 100 g/l K₂CO₃-Lösung gemäß Beispiel 1 modifiziert worden war. Nach 2 Stunden Reaktionszeit betrug die Dodecylamin-Ausbeute 95,9 % bei einem Umsatz von 99,9 %.

### Beispiel 24

Es wurde analog Beispiel 21 verfahren, jedoch mit der Abwandlung, dass der Raney-Nickel-Katalysator mit 200 g/l K₂CO₃-Lösung gemäß Beispiel 3 modifiziert worden war. Nach 2 Stunden Reaktionszeit betrug die Dodecylamin-Ausbeute 91,0 % bei einem Umsatz von 93,0 %.

### Beispiel 25

Es wurde analog Beispiel 21 verfahren, jedoch mit der Abwandlung, dass der Raney-Nickel-Katalysator mit 400 g/l K₂CO₃-Lösung gemäß Beispiel 4 modifiziert worden war. Nach 2 Stunden Reaktionszeit betrug die Dodecylamin-Ausbeute 68,3 % bei einem Umsatz von 69,9 %.

### Beispiel 26

Es wurde analog Beispiel 21 verfahren, jedoch mit der Abwandlung, dass der Raney-Nickel-Katalysator mit 150 g/l K₂CO₃-Lösung gemäß Beispiel 5 modifiziert worden war. Nach 4 Stunden Reaktionszeit betrug die Dodecylamin-Ausbeute 60,5 % bei einem Umsatz von 61,7 %.

### Beispiel 27

Es wurde analog Beispiel 21 verfahren, jedoch mit der Abwandlung, dass der Raney-Nickel-Katalysator mit 150 g/l K₂CO₃-Lösung gemäß Beispiel 6 modifiziert worden war. Nach 2 Stunden Reaktionszeit betrug die Dodecylamin-Ausbeute 95,8 % bei einem Umsatz von 99,8 %.

### Beispiel 28

Es wurde analog Beispiel 21 verfahren, jedoch mit der Abwandlung, dass der Raney-Nickel-Katalysator mit 150 g/l K₂CO₃-Lösung gemäß Beispiel 7 modifiziert worden war. Nach 2 Stunden Reaktionszeit betrug die Dodecylamin-Ausbeute 96,1 % bei einem Umsatz von 99,5 %.

### Beispiel 29

Es wurde analog Beispiel 21 verfahren, jedoch mit der Abwandlung, dass der Raney-Nickel-Katalysator mit 150 g/l K₂CO₃-Lösung gemäß Beispiel 8 modifiziert worden war. Nach 2 Stunden Reaktionszeit betrug die Dodecylamin-Ausbeute 95,4 % bei einem Umsatz von 99,9 %.

### Beispiel 30

Es wurde analog Beispiel 21 verfahren, jedoch mit der Abwandlung, dass der Raney-Nickel-Katalysator mit 143 g/l K₂CO₃-Lösung gemäß Beispiel 9 modifiziert worden war. Nach 2 Stunden Reaktionszeit betrug die Dodecylamin-Ausbeute 92,3 % bei einem Umsatz von 94,3 %.

### Beispiel 31

In einem Reaktor wurden 20 ml Lauronitril, 80 ml Cyclohexan und 1,4 g mit 150 g/l K₂CO₃-Lösung modifizierter Raney-Nickel-Katalysator vorgelegt. Es wurde unter 10 bar Wasserstoffdruck bei 125°C umgesetzt, wobei das Reaktionsgemisch mit 1500 UpM gerührt wurde. Der Lauronitril-Umsatz belief sich auf 99,6 % und die Dodecylamin-Ausbeute betrug 95,0 %.

### Beispiel 32

Es wurde analog Beispiel 21 verfahren, jedoch mit der Abwandlung, dass der Raney-Nickel-Katalysator mit 100 g/l KHCO₃-Lösung gemäß Beispiel 11 modifiziert worden war. Nach 1 Stunde Reaktionszeit betrug die Dodecylamin-Ausbeute 93,6 % bei einem Umsatz von 99,4 %.

### Beispiel 33

Es wurde analog Beispiel 21 verfahren, jedoch mit der Abwandlung, dass der Raney-Nickel-Katalysator mit 100 g/l Na₂CO₃-Lösung gemäß Beispiel 12 modifiziert worden war. Nach 1 Stunde Reaktionszeit betrug die Dodecylamin-Ausbeute 84,2 % bei einem Umsatz von 98,9 %.

### Beispiel 34

Es wurde analog Beispiel 21 verfahren, jedoch mit der Abwandlung, dass der Raney-Nickel-Katalysator mit 150 g/l Rb₂CO₃-Lösung gemäß Beispiel 13 modifiziert worden war. Nach 2 Stunden Reaktionszeit betrug die Dodecylamin-Ausbeute 96,3 % bei einem Umsatz von 99,7 %.

### Beispiel 35

Es wurde analog Beispiel 21 verfahren, jedoch mit der Abwandlung, dass der Raney-Nickel-Katalysator mit 150 g/l Cs₂CO₃-Lösung gemäß Beispiel 14 modifiziert worden war. Nach 2 Stunden Reaktionszeit betrug die Dodecylamin-Ausbeute 97,3 % bei einem Umsatz von 99,2 %.

### Beispiel 36

In dem Reaktor wurden 100 ml Laurinsäurenitril, 1 g mit 150 g/l K₂CO₃-Lösung modifizierter Nickelkatalysator auf Kieselgur/MgO (Beispiel 15) und 2 g (0,12 mol) Ammoniak vorgelegt. Es wurde unter 15 bar Wasserstoffdruck bei 125°C 3 Stunden umgesetzt, wobei das Reaktionsgemisch mit 1500 UpM gerührt wurde. Die Dodecylamin-Ausbeute betrug 99,2 % bei einem Umsatz von 99,5 %.

### Beispiel 37

In dem Reaktor wurden 100 ml Ölsäurenitril, 1 g mit 150 g/l K₂CO₃-Lösung modifizierter Nickelkatalysator auf Kieselgur/Al₂O₃ (Beispiel 16) und 2 g (0,12 mol) Ammoniak vorgelegt. Es wurde unter 10 bar Wasserstoffdruck bei 120°C 2 Stunden umgesetzt, wobei das Reaktionsgemisch mit 1500 UpM gerührt wurde. Die Ausbeute an primären Aminen (Oleylamin) betrug 99,3 % bei einem Umsatz von 99,7 %.

### Beispiel 38

In dem Reaktor wurden 100 ml Ölsäurenitril, 1 g mit 150 g/l K₂CO₃-Lösung modifizierter Cobaltkatalysator auf Kieselgur (Beispiel 17) und 3 g (0,12 mol) Ammoniak vorgelegt. Es wurde unter 60 bar Wasserstoffdruck bei 140°C 4 Stunden umgesetzt, wobei das Reaktionsgemisch mit 1500 UpM gerührt wurde. Die Ausbeute an primären Aminen (Oleylamin) betrug 99,6 % bei einem Umsatz von 99,8 %.

### Beispiel 39

In dem Reaktor wurden 100 ml Laurinsäurenitril, 1 g mit 150 g/l K₂CO₃-Lösung modifizierter Raney-Cobaltkatalysator (Beispiel 18) und 2 g (0,12 mol) Ammoniak vorgelegt. Es wurde unter 50 bar Wasserstoffdruck bei 160°C 4 Stunden umgesetzt, wobei das Reaktionsgemisch mit 1500 UpM gerührt wurde. Die Dodecylamin-Ausbeute betrug 98,8 % bei einem Umsatz von 99,2 %.

**Tabelle 2 Ergebnisse der Hydrierung**

| Beispiel | Katalysator | Ammoniak | Umsatz, % | Selektivität, % | Amin |
|---|---|---|---|---|---|
| 19 (V) | Raney-Nickel | nein | 99,7 | 83,2 | Dodecylamin |
| 20 (V) | Raney-Nickel | ja | 99,8 | 93,6 | Dodecylamin |
| 21 | Raney-Nickel | nein | 99,8 | 97,2 | Dodecylamin |
| 22 | Raney-Nickel | ja | 99,6 | 99,4 | Dodecylamin |
| 23 | Raney-Nickel | nein | 99,9 | 95,9 | Dodecylamin |
| 24 | Raney-Nickel | nein | 93,0 | 91,0 | Dodecylamin |
| 25 | Raney-Nickel | nein | 69,9 | 68,3 | Dodecylamin |
| 26 | Raney-Nickel | nein | 61,7 | 60,5 | Dodecylamin |
| 27 | Raney-Nickel | nein | 99,8 | 95,8 | Dodecylamin |
| 28 | Raney-Nickel | nein | 99,5 | 96,1 | Dodecylamin |
| 29 | Raney-Nickel | nein | 99,9 | 95,4 | Dodecylamin |
| 30 | Raney-Nickel | nein | 94,3 | 92,3 | Dodecylamin |
| 31 | Raney-Nickel | nein | 99,6 | 95,0 | Dodecylamin |
| 32 | Raney-Nickel | nein | 99,4 | 93,6 | Dodecylamin |
| 33 | Raney-Nickel | nein | 98,9 | 84,2 | Dodecylamin |
| 34 | Raney-Nickel | nein | 99,7 | 96,3 | Dodecylamin |
| 35 | Raney-Nickel | nein | 99,2 | 97,3 | Dodecylamin |
| 36 | Nickel | ja | 99,5 | 99,2 | Dodecylamin |
| 37 | Nickel | ja | 99,7 | 99,3 | Oleylamin |
| 38 | Cobalt | ja | 99,8 | 99,6 | Oleylamin |
| 39 | Raney-Cobalt | ja | 99,2 | 98,8 | Dodecylamin |

### Beispiele 40 bis 43

Es wurde die Aktivität bei wiederholter Verwendung von Raney-Nickel-Katalysator bei der Hydrierung von Lauronitril untersucht.

Im Autoklaven (Volumen 10 I) wurden 5 kg Lauronitril, 100 g Raney-Nickel-Katalysator und ggf. 250 g Ammoniak vorgelegt. Die Umsetzung wurde bei 10 bar Wasserstoff, 125°C und einer Rührgeschwindigkeit von 1000 UpM durchgeführt. Der Lauronitril-Umsatz und die Selektivität zu Dodecylamin wurden mit durch Recycling- und Probenahmeverluste abnehmenden Katalysatormengen bei verschiedenen Reaktionszeiten bestimmt. Es wurden Messungen mit modifiziertem und nicht modifiziertem Katalysator durchgeführt. Die Modifizierung des Katalysators erfolgte nach Beispiel 10.

**Tabelle 3: Hydrierung von Lauronitril mit nicht modifiziertem Katalysator und ohne Ammoniakzugabe (Beispiel 41)**

| Katalysatorverwendung Nr. | Menge | Reaktionszeit | Umsatz | Selektivität |
|---|---|---|---|---|
| | Katalysator g | h | % | % |
| 1 | 100 | 3,1 | 99,9 | 88,3 |
| 2 | 95 | 3,5 | 99,8 | 87,7 |
| 3 | 88 | 3,5 | 100 | 85,6 |
| 4 | 81 | 3,6 | 99,7 | 85,8 |
| 5 | 74 | 4,0 | 99,9 | 85,4 |

**Tabelle 4: Hydrierung von Lauronitril mit nicht modifiziertem Katalysator mit Zugabe von Ammoniak (Beispiel 42)**

| Katalysatorverwendung Nr. | Menge | Reaktionszeit | Umsatz | Selektivität |
|---|---|---|---|---|
| | Katalysator g | h | % | % |
| 1 | 100 | 3,7 | 100 | 97,6 |
| 2 | 94 | 5,2 | 92,9 | 93,7 |
| 3 | 84 | 6,1 | 85,7 | 94,5 |
| 4 | 73 | 7,8 | 92,4 | 96,0 |
| 5 | 65 | 9,5 | 73,2 | 95,9 |

**Tabelle 5: Hydrierung von Lauronitril mit modifiziertem Katalysator ohne Zugabe von Ammoniak (Beispiel 43)**

| Katalysatorverwendung Nr. | Menge | Reaktionszeit | Umsatz | Selektivität |
|---|---|---|---|---|
| | Katalysator g | h | % | % |
| 1 | 100 | 3,6 | 100 | 98,1 |
| 2 | 94 | 5,8 | 99,9 | 93,0 |
| 3 | 85 | 7,4 | 100 | 94,6 |
| 4 | 77 | 12,3 | 99,8 | 95,7 |
| 5 | 69 | 23,6 | 99,7 | 95,9 |

**Tabelle 6: Hydrierung von Lauronitril mit modifiziertem Katalysator und mit Ammoniakzugabe (Beispiel 44)**

| Katalysatorverwendung Nr. | Menge | Reaktionszeit | Umsatz | Selektivität |
|---|---|---|---|---|
| | Katalysator g | h | % | % |
| 1 | 100 | 3,5 | 99,6 | 99,9 |
| 2 | 94 | 8,1 | 99,2 | 99,9 |
| 3 | 88 | 10,6 | 99,1 | 99,7 |
| 4 | 79 | 11,8 | 99,5 | 98,7 |
| 5 | 70 | 10,8 | 100 | 99,4 |

## Patentansprüche

1. Verfahren zur Herstellung eines primären Amins durch Hydrierung von Nitrilen, bei dem man ein Reaktionsgemisch, das
(a) mindestens ein Nitril,
(b) Wasserstoff,
(c) gegebenenfalls Ammoniak und
(d) mindestens einen, durch Adsorption eines Alkalimetallcarbonats oder Alkalimetallhydrogencarbonats ex situ modifizierten, Cobalt- oder Nickelkatalysator enthält, welcher Alkalimetallcarbonat oder - hydrogencarbonat in einer Menge von 2 bis 12 Gew.-% enthält,
umsetzt.

2. Verfahren nach Anspruch 1, bei dem der modifizierte Katalysator mit Alkalimetallcarbonat oder Alkalimetallhydrogencarbonat durch Adsorption aus einer wässrigen Lösung mit einer Konzentration von 10 g/l bis 400 g/l hergestellt wird.

3. Verfahren nach Anspruch 2, bei dem der Katalysator mit einer wässrigen Lösung von K₂CO₃ mit einer Konzentration im Bereich von 50 bis 200 g/l modifiziert wird.

4. Verfahren nach einem oder mehreren der Ansprüche 1 bis 3, wobei es sich bei dem modifizierten Katalysator um einen modifizierten Raney-Nickel-Katalysator handelt.

5. Verfahren nach einem oder mehreren der Ansprüche 1 bis 4, wobei ein Nitril der Formel R-CN hydriert wird, in der R für eine Kohlenwasserstoffgruppe mit 1 bis 32 Kohlenstoffatomen steht.

6. Verfahren nach einem oder mehreren der Ansprüche 1 bis 5, bei dem der Katalysator im Reaktionsgemisch in der Menge von 1 bis 10 Gew.-%, bezogen auf das Nitril, vorliegt.

7. Verfahren nach einem oder mehreren der Ansprüche 1 bis 6, bei dem man die Nitrilhydrierung in Gegenwart von Ammoniak in der Menge von 1 bis 10 Gew.-%, bezogen auf das Nitril, durchführt.

8. Verfahren nach einem oder mehreren der Ansprüche 1 bis 7, bei dem man die Nitrilhydrierung in Anwesenheit von Cyclohexan durchführt.

9. Verfahren nach einem oder mehreren der Ansprüche 1 bis 8, bei dem man die Nitrilhydrierung unter einem Wasserstoffdruck von 1 bis 200 bar, insbesondere 2 bis 30 bar, durchführt.

10. Verfahren nach einem oder mehreren der Ansprüche 1 bis 9, bei dem man die Nitrilhydrierung im Temperaturbereich von 60 bis 250°C, insbesondere 100 bis 150°C, durchführt.

11. Modifizierter Cobalt- oder Nickelkatalysator, erhältlich durch Adsorption eines Alkalimetallcarbonats oder Alkalimetallhydrogencarbonats in einer Menge von 2 bis 12 Gew.-% auf einen üblichen Cobalt- oder Nickelkatalysator.

12. Katalysator nach Anspruch 11, wobei der Katalysator ein Raney-Nickel-Katalysator ist.

## Claims

1. A process for preparing a primary amine by hydrogenating nitriles, in which a reaction mixture which comprises
(a) at least one nitrile,
(b) hydrogen,
(c) optionally ammonia and
(d) at least one cobalt or nickel catalyst which contains alkali metal carbonate or hydrogencarbonate in an amount of from 2 to 12% by weight modified ex situ by adsorption of an alkali metal carbonate or alkali metal hydrogencarbonate
is converted.

2. The process as claimed in claim 1, in which the modified catalyst is prepared using alkali metal carbonates or alkali metal hydrogencarbonates by adsorption from an aqueous solution having a concentration of from 10 g/l to 400 g/l.

3. The process as claimed in claim 2, in which the catalyst is modified using an aqueous solution of K₂CO₃ having a concentration in the range from 50 to 200 g/l.

4. The process as claimed in one or more of claims 1 to 3, wherein the modified catalyst is a modified Raney nickel catalyst.

5. The process as claimed in one or more of claims 1 to 4, wherein a nitrile of the formula R-CN is hydrogenated in which R is a hydrocarbon group having from 1 to 32 carbon atoms.

6. The process as claimed in one or more of claims 1 to 5, in which the catalyst is present in the reaction mixture in an amount of from 1 to 10% by weight based on the nitrile.

7. The process as claimed in one or more of claims 1 to 6, in which the nitrile hydrogenation is carried out in the presence of ammonia in an amount of from 1 to 10% by weight based on the nitrile.

8. The process as claimed in one or more of claims 1 to 7, in which the nitrile hydrogenation is carried out in the presence of cyclohexane.

9. The process as claimed in one or more of claims 1 to 8, in which the nitrile hydrogenation is carried out under a hydrogen pressure of from 1 to 200 bar, in particular from 2 to 30 bar.

10. The process as claimed in one or more of claims 1 to 9, in which the nitrile hydrogenation is carried out in the temperature range of from 60 to 250°C, in particular from 100 to 150°C.

11. A modified cobalt or nickel catalyst obtainable by adsorption of an alkali metal carbonate or alkali metal hydrogencarbonate in an amount of from 2 to 12% by weight on a customary cobalt or nickel catalyst.

12. The catalyst as claimed in claim 11, which is a Raney nickel catalyst.

## Revendications

1. Procédé pour la préparation d'une amine primaire par hydrogénation de nitriles, dans lequel on transforme un mélange réactionnel qui contient
(a) au moins un nitrile,
(b) de l'hydrogène
(c) le cas échéant de l'ammoniac et
(d) au moins un catalyseur à base de nickel ou de cobalt modifié ex situ par l'adsorption d'un carbonate de métal alcalin ou d'un carbonate de métal alcalin qui contient du carbonate ou de l'hydrogénocarbonate de métal alcalin en une quantité de 2 à 12% en poids.

2. Procédé selon la revendication 1, dans lequel le catalyseur modifié par un carbonate de métal alcalin ou un hydrogénocarbonate de métal alcalin est préparé par adsorption à partir d'une solution aqueuse à une concentration de 10 g/l à 400 g/l.

3. Procédé selon la revendication 2, dans lequel le catalyseur est modifié avec une solution aqueuse de K₂CO₃ à une concentration dans la plage de 50 à 200 g/l.

4. Procédé selon l'une ou plusieurs des revendications 1 à 3, où il s'agit, pour le catalyseur modifié, d'un catalyseur modifié de type nickel de Raney.

5. Procédé selon l'une ou plusieurs des revendications 1 à 4, où on hydrogène un nitrile de formule R-CN, dans laquelle R représente un groupe hydrocarboné comprenant 1 à 32 atomes de carbone.

6. Procédé selon l'une ou plusieurs des revendications 1 à 5, dans lequel le catalyseur se trouve dans le mélange réactionnel en une quantité de 1 à 10% en poids, par rapport au nitrile.

7. Procédé selon l'une ou plusieurs des revendications 1 à 6, dans lequel on réalise l'hydrogénation de nitrile en présence d'ammoniac en une quantité de 1 à 10% en poids, par rapport au nitrile.

8. Procédé selon l'une ou plusieurs des revendications 1 à 7, dans lequel on réalise l'hydrogénation du nitrile en présence de cyclohexane.

9. Procédé selon l'une ou plusieurs des revendications 1 à 8, dans lequel on réalise l'hydrogénation de nitrile sous une pression d'hydrogène de 1 à 200 bars, en particulier de 2 à 30 bars.

10. Procédé selon l'une ou plusieurs des revendications 1 à 9, dans lequel on réalise l'hydrogénation de nitrile dans une plage de température de 60 à 250°C, en particulier de 100 à 150°C.

11. Catalyseur modifié à base de cobalt ou de nickel, pouvant être obtenu par adsorption d'un carbonate de métal alcalin ou d'un hydrogénocarbonate de métal alcalin en une quantité de 2 à 12% en poids sur un catalyseur usuel à base de cobalt ou de nickel.

12. Catalyseur selon la revendication 11, où le catalyseur est un catalyseur de type nickel de Raney.
